Europäisches Patentamt

European Patent Office

Office européen des brevets

(19)

(11) Veröffentlichungsnummer: **0 461 539 A2**

## (12) EUROPÄISCHE PATENTANMELDUNG

(21) Anmeldenummer: 91109183.3

(22) Anmeldetag: 05.06.91

(51) Int. Cl.⁵: **A61N 1/05**

(30) Priorität: 13.06.90 DE 4019002

(43) Veröffentlichungstag der Anmeldung:
**18.12.91 Patentblatt 91/51**

(84) Benannte Vertragsstaaten:
**DE FR GB IT NL SE**

(71) Anmelder: **Siemens Elema AB**
**Röntgenvägen 2**
**S-171 95 Solna 1(SE)**

(84) **SE**

Anmelder: **SIEMENS AKTIENGESELLSCHAFT**
**Wittelsbacherplatz 2**
**W-8000 München 2(DE)**

(84) **DE FR GB IT NL**

(72) Erfinder: **Heinze, Roland**
**Simbacher Strasse 9**
**W-8000 München 80(DE)**
Erfinder: **Stangl, Karl, Dr.**
**Denniger Strasse 150**
**W-8000 München 82(DE)**

(54) **Elektrodenanordnung für einen Defibrillator.**

(57) Um bei einer Elektrodenanordnung für einen Defibrillator (6) mit einer in einem Elektrodenträger (3) angeordneten Elektrode (2) zum flächigen Anlegen an der Außenseite des Herzens (1) eine definierte Anordnung eines oder mehrerer Sensoren (8) zum Herzen (1) zu ermöglichen, ist der Sensor (8) in dem Elektrodenträger (3) integriert und zur Steuerung des Defibrillators (6) über eine Sensorleitung (10) mit diesem verbunden.

FIG 1

EP 0 461 539 A2

Die Erfindung betrifft eine Elektrodenanordnung für einen Defibrillator mit einer in einem Elektrodenträger angeordneten flächenhaften Elektrode zum flächigen Anlegen an der Außenseite des Herzens und mit einer Elektrodenleitung zum verbinden der Elektrode mit dem Defibrillator.

Eine derartige Elektrodenanordnung ist aus der US-A-4030509 bekannt. Dabei ist die Elektrode flächenhaft ausgebildet und an der Außenseite des Herzens angeordnet, um im Falle einer kardialen Arrhythmie in Form einer Fibrillation möglichst alle erregbaren Zellen des Herzens durch eine von einem Defibrillator über eine Elektrodenleitung an die Elektrode abgegebenen Stromstoß depolarisieren zu können.

Zur Einleitung und Steuerung der Defibrillation ist es erforderlich, die Funktion des Herzens zu überwachen; dazu gehört insbesondere die Detektion von Tachykardien und Herzkammerflimmern. Dies geschieht bei bisher bekannten Defibrillatoren durch Erfassung von ausgewählten Herzparametern intrakardial im rechten Herzen. So ist bei einem aus der EP-A-0 009 255 bekannten Defibrillator ein Katheter u.a. mit Sensorelektroden im rechten Herzen plaziert, mit denen das innerkardiale EKG und indirekt über Impedanzmessung die mechanische Pumparbeit des Herzens erfaßt wird.

Aus der US-A-3 942 536 ist ein Kardiokonverter bekannt, bei dem ein im rechten Herzen plazierter Stimulationskatheter einen Drucksensor zur Überwachung der Herzfunktion aufweist.

Bei einem aus der US-A-3 805 795 bekannten Kardiokonverter ist ein Katheter vorgesehen, der Elektroden zur Erfassung des EKGs und ferner einen Sensor zur Erfassung der Muskelkontraktionen des Herzens aufweist; der Sensor besteht aus einem elastischen Kunststoffkörper mit darin eingebetteten Kohlepartikeln, deren Kontaktwiderstand sich bei Bewegung des Sensors ändert, so daß Muskelkontraktionen des Herzens durch Messung des Gesamtwiderstandes des Kunststoffkörpers erfaßbar sind.

Da der Einsatz intrakardialer Sensoren nur im rechten Herzen unproblematisch ist, kann mit den bekannten Sensorkathetern keine direkte Überwachung der Funktion des linken Herzens erfolgen.

Aus der US-A-4,763,646 ist zwar ein Herzschrittmacher mit einem oder mehreren Detektoren zur Überwachung von Herzgeräuschen bekannt, bei dem der als Mikrofon, Druckaufnehmer oder Beschleunigungsaufnehmer ausgebildete Detektor wahlweise in einer Elektrodenzuführung des Herzschrittmachers, innerhalb des Herzschrittmachergehäuses oder außerhalb des Herzschrittmachergehäuses mit diesem über eine eigene Zuleitung verbunden in der Nähe des Herzens angeordnet ist, jedoch ist die Anordnung der Detektoren außerhalb des Herzens undefiniert, so daß z.B. eine Unterscheidung zwischen Herzsignalen von dem rechten Herzen und Signalen vom linken Herzen nicht ohne weiteres möglich ist und eine Überlagerung der Signale durch andere Körperfunktionen nicht ausgeschlossen ist.

Der Erfindung liegt die Aufgabe zugrunde, in Verbindung mit einem Defibrillator eine definierte Anordnung eines oder mehrerer Sensoren zum Herzen zu ermöglichen, ohne daß dadurch der damit verbundene operative Aufwand erhöht wird.

Gemäß der Erfindung wird die Aufgabe dadurch gelöst, daß bei der Elektrodenanordnung der eingangs angegebenen Art in dem Elektrodenträger mindestens ein Sensor integriert ist und daß der Sensor zur Steuerung des Defibrillators über eine Sensorleitung mit diesen verbunden ist.

Mit der erfindungsgemäßen Elektrodenanordnung wird in vorteilhafter Weise die Möglichkeit genutzt, gleichzeitig mit der Applikation der Elektrode einen oder mehrere Sensoren direkt am Herzen anzubringen, um dessen Funktion überwachen zu können. Da der Sensor mittels der Elektrode direkt am Herzen fixiert wird, ist eine weitgehend störungsfreie Erfassung der Herzfunktion möglich, ohne daß die von dem Sensor zu erfassenden Herzparameter auf dem Wege vom Herzen zum Sensor durch zwischenliegende Muskel- oder Gewebsschichten geschwächt werden oder Störeinflüssen durch andere Körperfunktionen, wie z.B. der Atmung unterliegen. Ferner ist als Vorteil anzusehen, daß der Sensor in einer definierten, d.h. gegenüber der Herzgeometrie unveränderbaren Lage am Herzen fixiert ist, so daß insbesondere Bewegungsparameter des Herzens direkt und unverfälscht erfaßt werden. Dabei geht der zum Anbringen des Sensors erforderliche Aufwand nicht über den zum Applizieren der Elektrode an der Außenseite des Herzens ohnehin erforderlichen operativen Aufwand hinaus. Schließlich unterliegt der in dem Elektrodenträger integrierte Sensor im Vergleich zu den bisher intrakardial implantierten Sensoren keiner so engen Baugrößenbeschränkung, so daß die Verwendung größerer und damit im Prinzip auch empfindlicherer und genauerer Sensoren möglich ist.

Entsprechend vorteilhaften Weiterbildungen der erfindungsgemäßen Elektrodenanordnung ist der Sensor vorzugsweise ein Mikrofon, ein Druckaufnehmer, ein Beschleunigungsaufnehmer, ein Dehnungsmeßfühler oder ein Ultraschallsensor. Hierbei kommt insbesondere der Vorteil zum Tragen, daß der Sensor direkt am Herzen appliziert ist, so daß Bewegungs- und Geräuschparameter des Herzens unverfälscht von dem Sensor erfaßt werden. Weitere vorteilhafte Sensoren, die allein oder in Kombination zu den obengenannten Sensoren verwendet werden können sind ein optischer Sensor und eine Meßelektrode zur Partialdruckmessung von Sauerstoff, Kohlendioxid oder anderen Gaskonzentrationen im Herzmuskelgewebe. Der optische Sensor ist dabei vorzugsweise als Reflexionssensor mit einem nebeneinander liegenden Lichtsender und Lichtempfänger ausgebildet, wobei der Anteil des im Herzmuskelgewebe reflektierten Lichts vom Sauerstoffgehalt des Blutes im Herzmuskelgewebe abhängig ist. Der optische Sensor kann auch direkt über Blutgefäßen plaziert werden. Bei der Meßelektrode zur Partialdurckmessung kann die zugehörige Gegenelektrode aus dem Gehäuse des implantierten Defibrillators bestehen oder beispielsweise als Ringelektrode in dem Elektrodenträger integriert sein.

Bei einer vorteilhaften Weiterbildung der erfindungsgemäßen Elektrodenanordnung weist der Elektrodenträger zwei oder mehr strahlenförmig auseinanderlaufende Elektrodenarme auf, wobei in zumindest zwei Elektrodenarmen jeweils ein Sensor integriert ist. Dadurch wird es in vorteilhafter Weise ermöglicht, die Sensoren in genügend großem Abstand zueinander auf dem Herzen zu plazieren, so daß eine differenzierte Überwachung der Herzfunktion durch Bildung von Differenzsignalen der Sensoren möglich ist. Der gleiche Vorteil ergibt sich bei einer alternativen Ausführungsform der erfindungsgemäßen Elektrodenanordnung, bei der der Elektrodenträger aus zwei oder mehr Elektrodenköpfen besteht, wobei in zumindest zwei Elektrodenköpfen jeweils ein Sensor integriert ist. Dabei ergibt sich außerdem der Vorteil, daß das Herz großflächig mit der Elektrode und den Sensoren beaufschlagt werden kann, ohne daß dadurch die Hersbewegungen wesentlich beeinträchtigt werden.

Um bei mehreren in dem Elektrodenträger integrierten Sensoren eine Vielzahl von Sensorleitungen zwischen den Sensoren und dem Defibrillator zu vermeiden ist in vorteilhafter Weise in dem Elektrodenträger eine Auswahlschaltung integriert, die in Abhängigkeit von einer Steuerung durch den Defibrillator die Sensoren einzeln mit diesem über die Sensorleitung verbindet.

Zur Erhöhung der Übertragungssicherheit für die von den Sensoren erfaßten Herzparametern ist in dem Elektrodenträger in vorteilhafter Weise ein Meßverstärker für den oder die Sensoren integriert.

Zur Erläuterung der Erfindung wird im Folgenden auf die Figuren der Zeichnung Bezug genommen. Im einzelnen zeigen:

FIG 1    ein Ausführungsbeispiel für den prinzipiellen Aufbau der erfindungsgemäßen Elektrodenanordnung und deren Anordnung am menschlichen Herzen,

FIG 2    den in FIG 1 gezeigten Elektrodenträger in einer schnittdarstellung,

FIG 3    ein Ausführungsbeispiel für den in dem Elektrodenträger integrierten Sensor,

FIG 4    ein Beispiel für die schaltungstechnische Anordnung des Sensors in Bezug auf den Defibrillator, die

FIG 5,6,7    weiterer Ausführungsbeispiele für den Aufbau der erfindungsgemäßen Elektrodenanordnung und

FIG 8    ein weiteres Ausführungsbeispiel für die schaltungstechnische Anordnung mehrerer Sensoren in Bezug auf den Defibrillator.

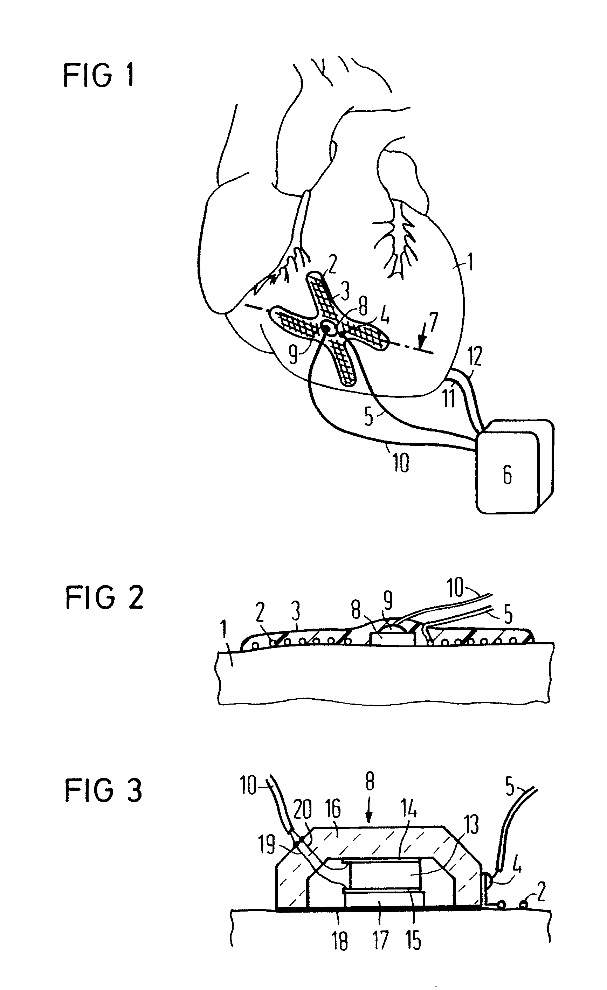

Das Ausführungsbeispiel nach FIG 1 zeigt den prinzipiellen Aufbau der erfindungsgemäßen Elektrodenanordnung und deren Anordnung am menschlichen Herzen (1). Die Elektrodenanordnung umfaßt eine flächenhafte Elektrode (2), die in einem Elektrodenträger (3) integriert ist und über diesen an der Außenseite des Herzens (1) an diesem flächig anliegend fixiert ist. Die Elektrode (2) ist an einer Anschlußstelle (4) über eine Elektrodenleitung (5) mit einem für eine Notfallmaßnahme außerhalb des Körpers oder bei einem dauergeschädigten Patient in dessen Körper implantierten Defibrillator (6) verbunden. Der Elektrodenträger (3) besteht aus einem flexiblen Isolierkörper, vorzugsweise Silikongummi, in dem die Elektrode (2) in Form eines Metallgitters derart eingebettet ist, daß die Elektrode (2), wie FIG 2 in einem (7) bezeichneten Schnitt durch den Elektrodenträger (3) und das darunterliegende Herzgewebe (1) zeigt, an der dem Herzen (1) zugewandten Seite des Elektrodenträgers (3) frei an der Außenwand des Herzens (1) zu liegen kommt und an der von dem Herzen (1) abgewandten Seite gegen den übrigen Körper durch den Elektrodenträger (3) isoliert ist. In dem flexiblen Elektrodenträger (3) ist ein Sensor (8) integriert, der an einer Anschlußstelle (9) über eine eigene Sensorleitung (10) mit dem Defibrillator (6) verbunden ist. Auf der von dem Applikationsort der Elektrode (2) abgewandten Seite des Herzens (1) ist - hier nicht sichtbar - eine weitere derartige Elektrode mit einem Sensor angeordnet, die ebenfalls über eine Elektrodenleitung (11) und Sensorleitung (12) mit dem Defibrillator (6) verbunden ist. Schließlich ist es möglich, daß der Defibrillator (6) auch mit Herzschrittmacherfunktionen ausgestattet ist und über eine intrakardial implantierbare Elektrode verfügt.

FIG 3 zeigt ein Ausführungsbeispiel für den Sensor (8), bei dem es sich um einen piezoelektrischen Meßwertaufnehmer handelt, der wahlweise als Mikrofon, Druckaufnehmer, Beschleunigungsaufnehmer oder Ultraschallsensor verwendbar ist. Der Sensor (8) weist eine piezokeramische Scheibe (13) auf, die zur Kontaktierung beidseitig mit zwei Metallschichten (14, 15) versehen ist und im Inneren eines topfförmigen Keramikkörpers (16) angeordnet ist. Dabei ist die mit (14) bezeichnete Metallschicht an dem Innenboden des topfförmigen Keramikkörpers (16) angeklebt, während die mit (15) bezeichnete Metallschicht über eine aus Isolierstoff bestehende Koppelmasse (17) mit einer den topfförmigen Keramikkörper (16) an seiner offenen Seite abschließenden Membran (18) verbunden ist. Der Sensor (8) ist derart in dem flexiblen Elektrodenträger (3) eingebettet, daß die Membran (18) an der für die Applikation auf dem Herzen (1) vorgesehenen Seite des Elektrodenträgers (3) frei liegt. Der Keramikkörper dient sowohl als Träger für die Anschlußstelle (9), an der die Metallschichten (14,15) mit Einzelleitungen (19,20) der Sensorleitung (10) verbunden sind, als auch als Träger für die Anschlußstelle (4), an der die Elektrode (2) mit der Elektrodenleitung (5) verbunden ist. Die Verwendung des Sensors (8) als Mikrofon, Druckaufnehmer, Beschleunigungsaufnehmer oder Ultraschallsensor hängt im wesentlichen von den Massen und Massenverhältnissen der Teile (16,17) sowie von der Art der Signalauswertung ab. So können in der Signalauswertung bestimmte parameterspezifische Frequenzanteile des aufgenommenen Signals durch Filter voneinander getrennt werden und parallel als beispielsweise Herzgeräusche (höherfrequent) oder Druck (niederfrequent) ausgewertet werden.

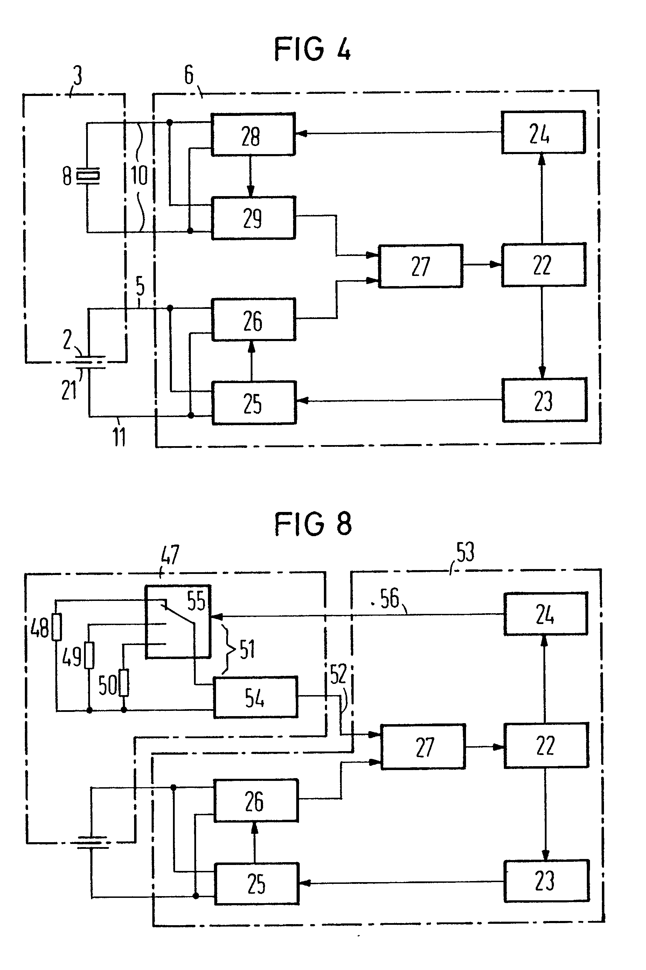

FIG 4 zeigt die schaltungstechnische Anordnung des Sensors (8) in Bezug auf den Defibrillator (6). Wie auch FIG 2 zeigt, umfaßt der auf das Herz (1) applizierbare Elektrodenträger (3) als elektrische Funktionsteile die Elektrode (2) und den Sensor (8). Der Elektrode (2) ist eine Gegenelektrode (21) zuzuordnen, die beispielsweise aus der in FIG 1 nicht sichtbaren Elektrode an der dem Applikationsort der Elektrode (2) abgewandten Seite des Herzens (1) oder einer in dem Herzen (1) angeordneten Katheterelektrode besteht. Der Defibrillator (6) umfaßt im wesentlichen eine zentrale Rechnereinheit (22), die eine der Elektrode (2) und der Gegenelektrode (21) zugeordnete Defibrillatorsteuereinheit (23) und eine dem Sensor (8) zugeordnete Sensorsteuereinheit (24) steuert. Die Defibrillatorsteuereinheit (23) steuert ihrerseits einen Impulsgenerator (25), der ausgangsseitig über die Elektrodenleitungen (5,11) mit der Elektrode (2) und der Gegenelektrode (21) verbunden ist und an diese entsprechend der Steuerung durch die Defibrillationssteuereinheit (23) Stromstöße mit einstellbarer Impulsamplitude und Impulsdauer abgibt. An der Elektrode (2) und der Gegenelektrode (21) ist ferner ein Meßverstärker (26) angeschlossen, der wahlweise eine Messung des EKGs oder der Herzimpedanz ermöglicht; bei Abgabe des Stromstoßes durch den Impulsgenerator (25) wird der Meßverstärker (26) von diesem für die entsprechende Zeitdauer abgeschaltet. Das Ausgangssignal des Meßverstärkers (26) wird der Rechnereinheit (22) über eine Signalverarbeitungseinheit (27) zugeführt, in der die gemessenen und verstärkten Signale in an sich bekannter Weise durch Filterung zur Weiterverarbeitung durch die Rechnereinheit (22) aufbereitet wird.

Die Sensorsteuereinheit (23) steuert einen Meßimpulsgenerator (28), der einen Betrieb des an diesen über die Sensorleitung (10) angeschlossenen Sensors (8) als Ultraschallgeber ermöglicht. Ein mit (29) bezeichneter weiterer Meßverstärker mißt die von dem Sensor (8) erfaßten Herzsignale und leitet diese verstärkt zur Weiterverarbeitung an die Signalverarbeitungseinheit (27) weiter. Die Rechnereinheit (22) steuert den Defibrillator (6) in Abhängigkeit von den über die Elektrode (2) und die Gegenelektrode (21) einerseits und den Sensor (8) andererseits erfaßten Meßsignale.

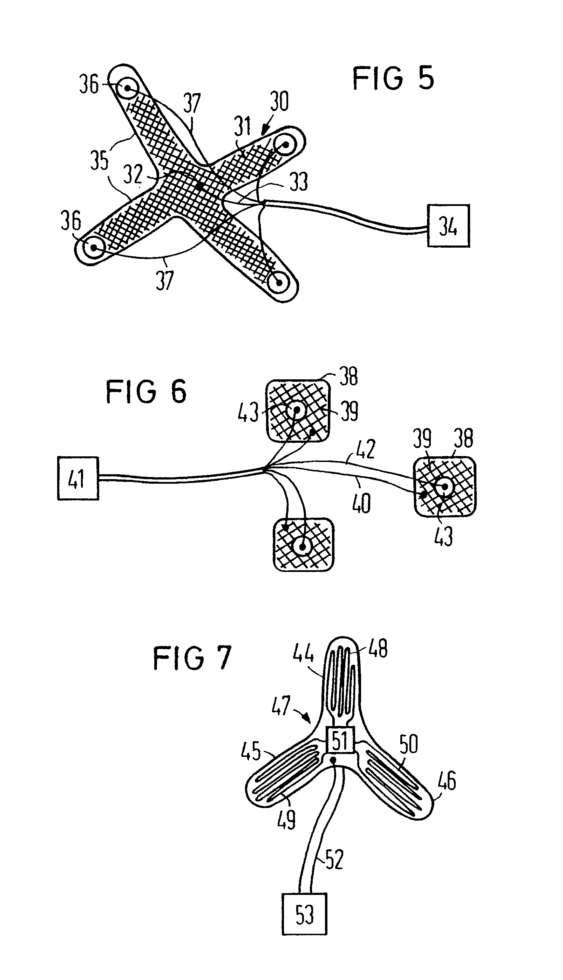

FIG 5 zeigt ein weiteres Ausführungsbeispiel für die erfindungsgemäße Elektrodenanordnung bestehend aus einem flexiblen Elektrodenträger (30) und einer darin eingebetteten gitterartigen Elektrode (31), die an einer Anschlußstelle (32) über eine Elektrodenleitung (33) mit einem Defibrillator (34) verbunden ist. Der Elektrodenträger (30) mit der Elektrode (31) weist mehrere strahlenförmig auseinanderlaufende Elektrodenarme (35) auf, in deren Endbereichen jeweils ein Sensor (36) integriert ist. Die Sensoren (36) sind jeweils über Sensorleitungen (37) mit dem Defibrillator (34) verbunden. Durch die strahlenförmige Anordnung der Elektrodenarme (35) wird erreicht, daß die Elektrode (31) großflächig am Herzen (1) (FIG 1) anliegt, ohne die Herzbewegungen wesentlich zu beeinträchtigen. Ferner liegen die Sensoren (36) jeweils relativ weit voneinander entfernt, so daß eine Erfassung von Parametern unterschiedlicher Herzbereiche ermöglicht ist.

Derselbe Vorteil wird bei einem alternativen Ausführungsbeispiel nach FIG 6 dadurch erreicht, daß der Elektrodenträger aus mehreren Elektrodenköpfen (38) mit Einzelelektroden (39) gebildet ist, die einzeln über Elektrodenleitungen (40) an einem Defibrillator (41) angeschlossen sind. Jeder Elektrodenkopf (38) trägt ferner einen über jeweils eine Sensorleitung (42) mit dem Defibrillator (41) verbundenen Sensor (43).

FIG 7 zeigt schließlich ein Ausführungsbeispiel für die erfindungsgemäße Elektrodenanordnung, bei dem in drei strahlenförmig auseinanderlaufenden Elektrodenarmen (44, 45,46) eines Elektrodenträgers (47) jeweils ein Dehnungsmeßfühler (48,49,50) als Sensor integriert ist. Die Dehnungsmeßfühler (48,49,50) sind von dem Isoliermaterial des Elektrodenträgers (47) jeweils vollständig umschlossen und gegenüber der hier nicht sichtbaren Elektrode in dem Elektrodenträger (47) isoliert. Die Sensoren (48,49,50) sind an einer gemeinsamen Auswahlschaltung mit einem Meßverstärker angeschlossen, die in Form einer integrierten Schaltung (51) ebenfalls in dem Elektrodenträger (47) integriert sind und über eine Sensorleitung (52) mit einem Defibrillator (53) verbunden sind.

FIG 8 zeigt die schaltungstechnische Anordnung der Sensoren (48,49,50) und des integrierten Schaltkreises (51) in Bezug auf den Defibrillator (53). Der Defibrillator (53) enthält, wie dies bereits in Verbindung mit FIG 4 gezeigt wurde, eine Rechnereinheit (22), eine Defibrillatorsteuereinheit (23), eine Sensorsteuereinheit (24) sowie einen der Elektrode und Gegenelektrode zugeordneten Impulsgenerator (25) und Meßverstärker (26). Der auf dem Elektrodenträger (47) angeordnete integrierte Schaltkreis (51) enthält einen Meßverstärker (54), der eingangsseitig über eine Auswahlschaltung (55) mit den Sensoren (48, 49, 50) verbunden ist. Der Meßverstärker (54) ist ausgangsseitig über die Sensorleitung (52) mit der Signalverarbeitungseinheit (27) in dem Defibrillator (53) verbunden, während die Auswahlschaltung (53) über eine Steuerleitung (56) als Bestandteil der Sensorleitung (52) mit der Sensorsteuereinheit (24) in dem Defibrillator (53) verbunden ist.

Bezugszeichenliste

| | |
|---|---|
| 1 | Herz |
| 2 | Elektrode |
| 3 | Elektrodenträger |
| 4 | Anschlußstelle für 2 |
| 5 | Elektrodenleitung |
| 6 | Defibrillator |
| 7 | Schnitt durch 3 |
| 8 | Sensor |
| 9 | Anschlußstelle für 8 |
| 10 | Sensorleitung |
| 11 | Elektrodenleitung |
| 12 | Sensorleitung |
| 13 | piezokeramische Scheibe |
| 14,15 | Metallschichten |
| 16 | Keramikträger |
| 17 | Koppelmasse |
| 18 | Membran |
| 19,20 | Einzelleitungen |
| 21 | Gegenelektrode |
| 22 | Rechnereinheit |
| 23 | Defibrillatorsteuereinheit |
| 24 | Sensorsteuereinheit |
| 25 | Impulsgenerator |
| 26 | Meßverstärker |
| 27 | Signalverarbeitungseinheit |
| 28 | Meßimpulsgenerator |
| 29 | weiterer Meßverstärker |
| 30 | Elektrodenträger |
| 31 | Elektrode |
| 32 | Anschlußstelle von 31 |
| 33 | Elektrodenleitung |

| | |
|---|---|
| 34 | Defibrillator |
| 35 | Elektrodenarme von 30 |
| 36 | Sensor |

Bezugszeichenliste

| | |
|---|---|
| 37 | Sensorleitung |
| 38 | Elektrodenköpfe |
| 39 | Einzelelektroden |
| 40 | Elektrodenleitungen |
| 41 | Defibrillator |
| 42 | Sensorleitung |
| 43 | Sensor |
| 44,45,46 | Elektrodenarme von 47 |
| 47 | Elektrodenträger |
| 48,49,50 | Dehnungsmeßfühler |
| 51 | integrierte Schaltung |
| 52 | Sensorleitung |
| 53 | Defibrillator |
| 54 | Meßverstärker |
| 55 | Auswahlschaltung |
| 56 | Steuerleitung |

**Patentansprüche**

1. Elektrodenanordnung für einen Defibrillator (6) mit einer in einem Elektrodenträger (3) angeordneten flächenhaften Elektrode (2) zum flächigen Anlegen an der Außenseite des Herzens (1) und mit einer Elektrodenleitung (5) zum Verbinden der Elektrode (2) mit dem Defibrillator (6), **dadurch gekennzeichnet,** daß in dem Elektrodenträger (3) mindestens ein Sensor (8) integriert ist und daß der Sensor (8) zur Steuerung des Defibrillators (6) über eine Sensorleitung (10) mit diesem verbunden ist (FIG 1).

2. Elektrodenanordnung nach Anspruch 1, **dadurch gekennzeichnet,** daß der Sensor (8) ein Mikrofon ist.

3. Elektrodenanordnung nach Anspruch 1, **dadurch gekennzeichnet,** daß der Sensor (8) ein Druckaufnehmer ist.

4. Elektrodenanordnung nach Anspruch 1, **dadurch gekennzeichnet,** daß der Sensor (8) ein Beschleunigungsaufnehmer ist.

5. Elektrodenanordnung nach Anspruch 1, **dadurch gekennzeichnet,** daß der Sensor (8) ein Ultraschallsensor ist.

6. Elektrodenanordnung nach Anspruch 1, **dadurch gekennzeichnet,** daß der Sensor (48,49,50) ein Dehnungsmeßfühler ist (FIG 7).

7. Elektrodenanordnung nach Anspruch 1, **dadurch gekennzeichnet,** daß der Sensor ein optischer Sensor ist.

8. Elektrodenanordnung nach Anspruch 1, **dadurch gekennzeichnet,** daß der Sensor eine Meßelektrode zur Partialdruckmessung von Gasen ist.

9. Elektrodenanordnung nach einem der vorangehenden Ansprüche, **dadurch gekennzeichnet,** daß der Elektrodenträger (30) zwei oder mehr strahlenförmig auseinanderlaufende Elektrodenarme (35) aufweist und daß in zumindest zwei Elektrodenarmen (35) jeweils ein Sensor (36) integriert ist (FIG 5).

10. Elektrodenanordnung nach einem der Ansprüche 1 bis 8, **dadurch gekennzeichnet,** daß der Elektrodenträger aus zwei oder mehr Elektrodenköpfen (38) besteht, und daß in zumindest zwei Elektrodenköpfen (38) jeweils ein Sensor (43) integriert ist (FIG 6).

11. Elektrodenanordnung nach einem der vorangehenden Ansprüche, **dadurch gekennzeichnet,** daß bei mehreren, in dem Elektrodenträger (47) integrierten Sensoren (48,49,50) in diesem außerdem eine Auswahlschaltung (55) integriert ist, die in Abhängigkeit von einer Steuerung durch den Defibrillator (53) die Sensoren (48,49,50) einzeln mit dem Defibrillator (53) über die Sensorleitung (52) verbindet (FIG 7 und 8).

12. Elektrodenanordnung nach einem der vorangehenden Ansprüche, **dadurch gekennzeichnet,** daß in dem Elektrodenträger (47) ein Meßverstärker (54) für den mindestens einen Sensor (48,49,50) integriert ist (FIG 7 und 8).

FIG 1

FIG 2

FIG 3

## FIG 4

## FIG 8

FIG 5

FIG 6

FIG 7